# EUROPÄISCHE PATENTANMELDUNG

(11) **EP 1 405 847 A1**
(43) Veröffentlichungstag der Anmeldung: **07.04.2004**
(21) Anmeldenummer: 03020132.1
(22) Anmeldetag: 05.09.2003
(51) Int. Cl.: C07D 213/89

(54) **Verfahren zur Herstellung von substituierten Pyridin-N-oxid-verbindungen**

(30) Priorität: 01.10.2002 AT 14822002
(71) Anmelder: DSM Fine Chemicals Austria Nfg GmbH & Co KG, 4021 Linz (AT)
(72) Erfinder: Zich, Thomas, 4020 Linz (AT); Schiek, Wolfgang, 94032 Passau (DE); Rössler, Markus, 4030 Linz (AT); Seifter, Christian, 4030 Linz (AT)
(74) Vertreter: Lindinger, Ingrid

(57) **Zusammenfassung**

Verfahren zur Herstellung von substituierten Pyridin-N-oxid-verbindungen der Formel 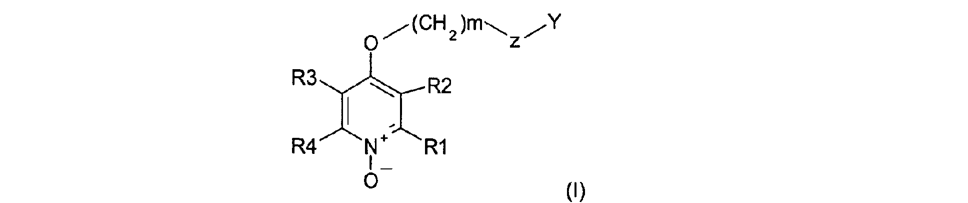 in der R1, R2, R3 und R4 H, eine Carboxylgruppe oder ein C₁-C₁₂-Alkylrest, der Atome aus der Gruppe N, O, oder S enthalten kann, sein können, oder R1 und R2 und/oder R3 und R4 gemeinsam einen gegebenenfalls substituierten C₄-C₂₀-Alkylenrest bilden können, der Atome aus der Gruppe N, O, oder S enthalten kann, A Benzyl oder eine Gruppe (CH₂)ₘ bedeutet, wobei
m eine ganze Zahl von 1 bis 12 sein kann,
Z₁ und Z₂ unabhängig voneinander O oder S sind und Y H, einen C₁-C₁₂- Alkylrest, der gegebenenfalls Atome aus der Gruppe N, O, oder S enthalten kann, einen C₆-C₂₀-Arylrest oder einen C₅-C₂₀-Heterocyclus bedeutet, wobei die Reste gegebenenfalls substituiert sein können,
oder Z₂ und Y gemeinsam einen gegebenenfalls substituierten Ring bzw. Ringsystem bilden, wobei der Ring oder das Ringsystem Atome aus der Gruppe N, O, oder S enthalten kann, aus dem korrespondierenden 4-Halo-Pyridin-N-oxid der Formel 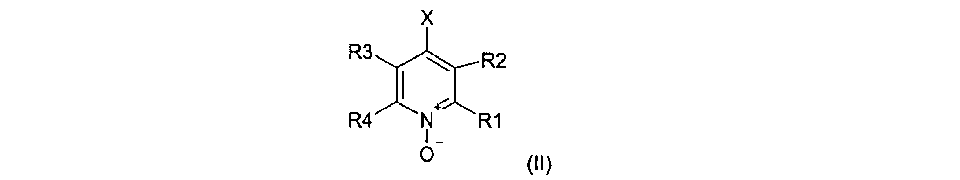 in der X Chlor, Brom oder lod bedeutet, wobei die Verbindung der Formel (II) in Gegenwart eines Phasentransferkatalysators und einer Base mit einer Verbindung der Formel

HZ₁-A-Z₂-Y (III)

in der Z₁, Z₂, A und Y wie oben definiert sind, bei einer Temperatur bis zur Rückflusstemperatur zu der entsprechenden substituierten Pyridin-N-oxid-Verbindung der Formel (I) umgesetzt wird, sowie ein Verfahren zur Herstellung der Verbindung der Formel (II).

## Beschreibung

Die Erfindung betrifft ein Verfahren zur Herstellung von substituierten Pyridin-N-oxid-verbindungen mittels Phasentransferkatalyse.

Substituierte Pyridin-N-oxid-verbindungen werden in der pharmazeutischen Industrie als Zwischenverbindungen bei der Herstellung von Arzneimitteln eingesetzt, die beispielsweise gegen Heliobacter-Bakterien wirksam sind oder etwa zur Behandlung und Vorbeugung von Magengeschwüren verwendet werden.

Die Herstellung substituierter Pyridin-N-oxid-verbindungen aus den entsprechenden Chloroderivaten ist beispielsweise aus EP-A-0 268 956, WO 98/28299 oder WO 96/02534 bekannt. Die in diesen Literaturstellen beschriebenen Synthesen erfordern jedoch die Verwendung einer starken Base wie etwa Natriumhydrid in unterschiedlichen Lösungsmitteln, wie DMF, DMSO oder NMP oder die direkte Herstellung eines Natriumalkoholats aus metallischem Natrium und Alkohol. Weiters erzielen sie zumeist mäßige Ausbeuten.
Gemäß diesem Stand der Technik ist die Herstellung der gewünschten substituierten Pyridin-N-oxid-verbindungen auch ohne Verwendung eines Lösungsmittels möglich. Die oben genannten Nachteile, im speziellen die Verwendung starker Basen, das daraus resultierende Gefahrenpotential und die notwendigen Maßnahmen für ein sicheres Handling dieser Substanzen, die Notwendigkeit, wenn überhaupt, dann trockene Lösungsmittel zu verwenden, das Entstehen von Wasserstoffgas, die mangelnde Selektivität der Umsetzung, sowie oftmals erforderliche Parameter wie hohe Temperatur oder lange Reaktionszeit werden dadurch nicht aufgehoben.
Aus EP 0 297 783 ist die Herstellung von Alkoxypyridin-1-oxid-verbindungen aus den entsprechenden Nitroderivaten bekannt. Dabei werden die entsprechenden Nitropyridin-1-oxid-verbindungen mit einem einfachen Alkohol oder einem Alkoholat der Formel ROM, in der M gleich H oder ein Alkalimetall ist, in Gegenwart einer Base und eines Phasentransferkatalysators umgesetzt.

Die Umsetzung von Chloroderivaten mit Diolen, Mercaptoalkoholen, Etheralkoholen, Thioetheralkoholen, Dithiolen oder Thioetherthiolen, die zu den aus EP-A-0 268 956, WO 98/28299 oder WO 96/02534 bekannten substituierten Pyridin-N-oxid-verbindungen führen, erfolgte jedoch bisher analog den vorher beschriebenen Verfahren.

Es bestand die Aufgabe, die Reaktion dergestalt zu modifizieren, dass die verwendeten starken Basen durch eine kostengünstigere Alternative ersetzt werden, die weder brennbar noch explosiv ist, die Verwendung der schwer rezyklierbaren Lösungsmittel, wie DMF, DMSO zu vermeiden und eine Umsetzung in guter Selektivität im mindestens vergleichbarer Zeit und bei vergleichbaren Temperaturen zu erreichen.

Unerwarteterweise konnte diese Aufgabe durch die Verwendung von Phasentransferkatalysatoren gelöst werden.

Gegenstand der Erfindung ist demnach ein Verfahren zur Herstellung von substituierten Pyridin-N-oxid-verbindungen der Formel 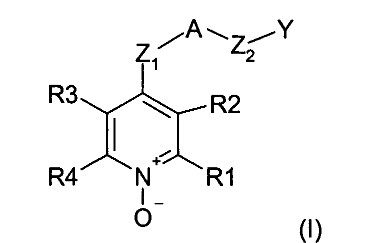 in der R1, R2, R3 und R4 unabhängig voneinander H, eine Carboxylgruppe oder ein C₁-C₁₂-Alkylrest, der gegebenenfalls ein oder mehrere Atome aus der Gruppe N, O, oder S enthalten kann, sein können, oder R1 und R2 und/oder R3 und R4 gemeinsam einen gegebenenfalls substituierten C₄-C₂₀-Alkylenrest bilden können, der ein oder mehrere Atome aus der Gruppe N, O, oder S enthalten kann, A Benzyl oder eine Gruppe (CH₂)ₘ bedeutet, wobei
m eine ganze Zahl von 1 bis 12 sein kann,
Z₁ und Z₂ unabhängig voneinander O oder S sind und Y H, einen C₁-C₁₂- Alkylrest, der gegebenenfalls ein oder mehrere Atome aus der Gruppe N, O, oder S enthalten kann, einen C₆-C₂₀-Arylrest oder einen C₅-C₂₀-Heterocyclus bedeutet, wobei die Reste gegebenenfalls durch Halogen, C₁-C₆-Alkyl, Nitro, Phenyl oder tert. Amin substituiert sein können,
oder Z₂ und Y gemeinsam einen gegebenenfalls substituierten Ring oder ein gegebenenfalls substituiertes Ringsystem bilden, wobei der Ring oder das Ringsystem ein oder mehrere Atome aus der Gruppe N, O, oder S enthalten kann,
aus dem korrespondierenden 4-Halo-Pyridin-N-oxid der Formel 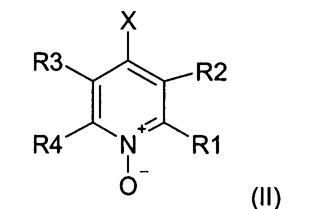 in der R1-R4 wie oben definiert sind und X Chlor, Brom oder Iod bedeutet, das dadurch gekennzeichnet ist, dass die Verbindung der Formel (II) in Gegenwart eines Phasentransferkatalysators und einer Base mit einer Verbindung der Formel

HZ₁-A-Z₂-Y (III)

in der Z₁, Z₂, A und Y wie oben definiert sind, bei einer Temperatur bis zur Rückflusstemperatur zu der entsprechenden substituierten Pyridin-N-oxid-Verbindung der Formel (I) umgesetzt wird.

Gemäß vorliegender Erfindung werden substituierte Pyridin-N-oxid-Verbindungen der Formel (I) hergestellt.
In der Formel (I) bedeuten R1, R2, R3 und R4 unabhängig voneinander H, eine Carboxylgruppe oder einen C₁-C₁₂-Alkylrest, der gegebenenfalls ein oder mehrere Atome aus der Gruppe N, O, oder S enthalten kann.

Unter C₁-C₁₂-Alkylresten sind dabei lineare, verzweigte oder cyclische Alkylreste, wie etwa Methyl, Ethyl, n-Propyl, i-Propyl, n-Butyl, sek.-Butyl, tert. Butyl, n-Pentyl, Hexyl, Cyclohexyl u.s.w. zu verstehen.
Die Alkylkette kann dabei ein oder mehrere Atome aus der Gruppe N, S oder O enthalten.

R1 und R2 und/oder R3 und R4 können aber auch gemeinsam einen gegebenenfalls substituierten C₄-C₂₀-Alkylenrest bilden, der ein oder mehrere Atome aus der Gruppe N, O, oder S enthalten kann.
Der Alkylenrest kann weiters noch, in Abhängigkeit von der Größe des gebildeten Ringes, noch ein oder zwei Doppelbindungen aufweisen.

Bevorzugt bedeuten R1, R2, R3 und R4 unabhängig voneinander H oder C₁-C₄-Alkyl, besonders bevorzugt H oder Methyl.

A kann entweder Benzyl oder eine (CH₂)ₘ-Gruppe sein, wobei m eine ganze Zahl von 1 bis 12, bevorzugt eine Zahl von 2 bis 6 bedeutet.
Z₁ und Z₂ können unabhängig voneinander O oder S sein und Y kann H, einen C₁-C₁₂- Alkylrest, der gegebenenfalls ein oder mehrere Atome aus der Gruppe N, O, oder S enthalten kann, einen C₆-C₂₀-Arylrest oder einen C₅-C₂₀-Heterocyclus bedeuten, wobei die Reste gegebenenfalls durch Halogen, C₁-C₆-Alkyl, Nitro, Phenyl oder tert. Amin substituiert sein können.

Unter C₆-C₂₀-Aryl sind beispielsweise Verbindungen, wie etwa Phenyl, Biphenyl, Naphthyl u.s.w. zu verstehen

Unter Heterocyclus sind cyclische Reste zu verstehen die zumindestens ein S-, O-oder N-Atom im Ring enthalten. Dies sind beispielsweise Furyl, Pyridyl, Pyrimidyl, Thienyl, Isothiazolyl, Imidazolyl, Tetrazolyl, Pyrazinyl, Benzofuranyl, Benzothiophenyl, Chinolyl, Isochinolyl, Benzothienyl, Isobenzofuryl, Pyrazolyl, Indolyl, Isoindolyl, Benzoimidazolyl, Purinyl, Carbazolyl, Oxazolyl, Thiazolyl, 1,2,4-Thiadiazolyl, Isoxazolyl, Pyrrolyl, Chinazolinyl, Pyridazinyl, Phthalazinyl, Morpholinyl, u.s.w.

Z₂ und Y können aber auch gemeinsam einen gegebenenfalls substituierten Ring oder ein gegebenenfalls substituiertes Ringsystem bilden, wobei der Ring oder das Ringsystem ein oder mehrere Atome aus der Gruppe N, O, oder S enthalten kann.
Beispiele dafür sind 3-Nitro[1,2]b-pyridazin, 4-Methylthiazol, Methyltriazol, Imidazol, u.s.w.

Bevorzugt bedeutet Y H, einen C₁-C₆-Alkylrest, der ein oder zwei Atome aus der Gruppe O oder S enthalten kann, einen C₆-C₁₀-Aryl- oder C₅-C₁₀-Heterocyclusrest, wobei die Reste gegebenenfalls durch C₁-C₄-Alkyl, Halogen, Nitro, tert. Amin oder Phenyl substituiert sein können.

Beispiele für Verbindungen der Formel (I), die durch das erfindungsgemäße Verfahren hergestellt werden können sind etwa 4-(2-Benzyloxyethoxy)-2,3-dimethyl-pyridin-N-oxid, 4-(3-methoxypropoxy)-2,3-dimethyl-pyridin-N-oxid, 4-(2-Hydroxyethoxy)-2,3-dimethyl-pyridin-N-oxid, 4-(4-Methoxybutoxy)-2-methyl-pyridin-N-oxid, 4-(3-Methoxypropoxy)-2-methyl-pyridin-N-oxid, 4-(3-Methoxypropoxy)-2,3,5-dimethylpyridin-N-oxid, 2,3-Dimethyl-4-(3-hydroxypropylthio)pyridin-N-oxid, 2,3-Dimethyl-4-(2-hydroxyethyl-thio)pyridin-N-oxid, 2,3-Dimethyl-4-(3-hydroxypropylthio)pyridin-N-oxid, 4**(?)**,6-Dimethyl-4-(3-hydroxypropylthio)pyridin-N-oxid, u.s.w..

Als Ausgangsverbindung dient eine entsprechende 4-Halo-Pyridin-N-oxid-Verbindung der Formel (II), in der R1-R4 wie in der Formel (I) definiert sind und X Chlor, Iod oder Brom bedeutet.
Bevorzugt bedeutet X Chlor.

Geeignete Verbindungen der Formel (II) sind beispielsweise 4-Chlor-2-methylpyridin-N-oxid, 4-Chlor-2,3,5-trimethylpyridin-N-oxid, 4-Chlor-2,3,5,6-Tetramethylpyridin-N-oxid, 4-Chlor-2,5-dimethylpyridin-N-oxid, 4-Brom-3,5-dimethylpyridin-N-oxid u.s.w..

Die Verbindung der Formel (II) kann beispielsweise aus der entsprechenden 4-Nitropyridin-N-oxid-Verbindung, etwa analog EP 0 268 956 durch Umsetzung mit Acetylchlorid bei -10°C hergestellt werden. Eine weitere Herstellvariante stellt das Erhitzen mit konzentrierter Salzsäure im Autoklaven bei 170°C dar, wie etwa in J. Med. Chem. 1998, 41,1777 beschieben.
Die Verbindung der Formel (II) kann aber auch durch Umsetzung der korrespondierenden 4-Nitropyridin-N-oxid-Verbindung in Gegenwart eines Phasentransferkatalysators und in Anwesenheit einer Säure mit einem entsprechenden Salz in einem organischen Lösungsmittel hergestellt werden.
Diese Herstellvariante ist neu und somit auch Gegenstand der vorliegenden Erfindung.
Dabei werden 4-Nitropyridin-N-oxid-Verbindungen der Formel 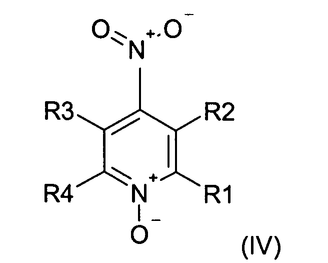 in der R1 bis R4 wie in der Formel (I) definiert sind in Gegenwart eines Phasentransferkatalysators und in Anwesenheit einer Säure mit einem geeigneten, gut löslichen Salz umgesetzt.
Als Salze eignen sich beispielsweise Alkalihalogenide, wie etwa Alkalichloride, - bromide oder -iodide.

Das Salz wird dabei im molaren Überschuss, bezogen auf die Nitroverbindung, eingesetzt. Bevorzugt wird ein molares Verhältnis von Verbindung der Formel (IV) : Salz von 1: 1,5 bis 1: 5, besonders bevorzugt von 1:2 bis 1:4.

Als Lösungsmittel eignen sich gegebenenfalls halogenierte Kohlenwasserstoffe, wie etwa Dichlormethan, Toluol, u.s.w.; Ether, wie etwa Diisopropylether u.s.w., oder Acetonitril

Die Umsetzung erfolgt erfindungsgemäß in Gegenwart eines Phasentransferkatalysators. Als Phasentransferkatalysatoren kommen für das erfindungsgemäße Verfahren quartäre Ammoniumsalzverbindungen der Formel (V) R₅R₆R₇R₈N⁺A⁻ in Frage, wobei die Reste R₅ bisR₈ unabhängig voneinander C₁-C₂₀-Alkyl, Phenyl, Arylalkyl oder Alkylaryl bedeuten können. Die Reste können dabei auch gegebenenfalls einoder mehrfach durch funktionelle Gruppen, beispielsweise durch Amin- oder Alkoholgruppen oder durch C₁-C₄-Alkoxy substituiert sein.
Unter C₁-C₂₀-Alkyl sind dabei lineare, verzweigte oder cyclische Reste, wie etwa Methyl, Ethyl, n-Propyl, 1-Propyl, n-Butyl, sek.-Butyl, tert. Butyl, n-Pentyl, Hexyl, Cyclohexyl, Octyl, Undecyl, Dodecyl, Hexadecyl, u.s.w. zu verstehen.
Unter Arylalkyl ist dabei beispielsweise Tolyl zu verstehen. Alkylaryl bezieht sich auf Alkylgruppen, die einen Arylsubstituenten aufweisen, wie etwa Benzyl.

R₅ und R₆ können aber auch zu einem 5- oder 6-gliedrigem, heterocyclischen System verknüpft sein, das mindestens ein quaternisiertes Stickstoffatom und gegebenenfalls weitere Atome aus der Gruppe N, S oder O enthält. Ein Beispiel dafür ist 1-(Ethylhexyl)-4-dimethylamin-pyridin (EtHexDMAP).

Bevorzugt sind Verbindungen der Formel (V) in der R₅-R₈ unabhängig voneinander einen C₁-C₁₆-Alkylrest oder Benzylrest bedeuten.

A⁻ bedeutet in der Formel (V) ein Anion, das nach Belieben angepasst werden kann. Beispiele für geeignete Anionen sind etwa Chlorid, Bromid, Fluorid, Iodid, Hydroxid, Hydrogensulfat, Perchlorat, Nitrat, Acetat, Benzoat, Mesylat, u.s.w..
Bevorzugt bedeutet A- Chlorid oder Bromid.

Beispiele für geeignete Verbindungen der Formel (V) sind etwa Tetraalkylammoniumsalze, wie Tetraethyl-, Tetrapropyl-, Tetrabutyl- bis Tetradodecylammoniumchloride oder -bromide, Tributylhexadecylammoniumchlorid oder -bromid, Trioctylmethyl-(Aliquat 336), Tributylmethyl-, Tridecylmethylammoniumchlorid, -bromide oder - hydrogensulfate u.s.w..; Arylalkylammoniumsalze, wie Tetrabenzyl-, Benzyltrimethyl-, Benzyltriethyl-, Benzyltributylammoniumchloride oder -bromide u.s.w.;
Arylammoniumsalze, wie Triphenylmethylammoniumchlorid, -fluorid oder -bromid, N,N,N-Trimethylanilliniumbromid, N,N-Diethyl-N-ethylanillinium-hydrogen-sulfat, Trimethylnaphthylammoniumchlorid, 5- und 6-gliedrige, heterocyclische Verbindungen, die zumindest ein quarternisiertes Stickstoffatom im Ring tragen, wie z.B. N-Methylpyridiniumchlorid, N-Hexylpyridiniumiodid, 4-Pyridyltrimethylammoniumiodid, 1-Methyl-1-azabicyclo[2.2.1]heptanbromid, N,N-Dibutylmorpholiniumchlorid, N-Ethylthiazoliumchlorid, N-Butylpyrroliumchlorid und andere Substanzen.
Für das erfindungsgemäße Verfahren geeignete Phasentransferkatalysatoren sind aber auch der obigen Beschreibung entsprechende quartäre Phosphoniumsalze, sowie Polyether, cyclischer oder acyclischer Natur, wie PEG's und Kronenether oder Tris-[2-(2-methoxyethoxy)-ethyl]-amin (TDA-1) sowie alkylsubstituierte Guanidiniumsalze.

Bevorzugte Phasentransferkatalysatoren für die Umsetzung der Nitroverbindung der Formel (IV) zu der korrespondierenden Haloverbindung der Formel (II) sind Tetrabutylammoniumsalze, Tributylhexadecylammoniumsalze, Benzyltributylammoniumsalze, Trioctylmethylammoniumsalze, Tridecylmethylammoniumsalze, Triethylmethylammoniumsalze, sowie Tributylmethylammoniumsalze oder deren Gemische.

Besonders bevorzugt sind die Chloride und Bromide der oben angeführten Ammoniumsalze.

Als Säure eignen sich unter den Reaktionsbedingungen nicht oxidierende organische oder anorganische Säuren, wie etwa HCl, HBr oder Eisessig.

Die Umsetzung erfolgt bevorzugt bei Rückflusstemperatur.

Nach erfolgter Umsetzung wird die entsprechende Verbindung der Formel (II) extraktiv aus dem Reaktionsgemisch isoliert und sodann für die Umsetzung zu dem gewünschten Endprodukt der Formel (I) verwendet.

Die Verbindung (II) wird mit einem Alkohol oder Thiol der Formel (III) umgesetzt. In der Formel (III) sind A, Z₁, Z₂ und Y wie in Formel (I) definiert.

Geeignete Alkohole oder Thiole der Formel (III) sind demnach beispielsweise 3-Methoxypropanol, 2-Benzyloxyethanol, Ethylenglycol, 3-(3-Methoxypropoxy)-propanol, 4-Methoxybutanol, 4-Ethoxybutanol, 3-Methylthiopropanthiol, 3-Hydroxymercaptan, Ethandithiol u.s.w..

Die beiden Ausgangsverbindungen der Formel (II) und der Formel (III) werden bei dem erfindungsgemäßen Verfahren in einem Molverhältnis von 1:1,1 bis 1:10, bevorzugt von 1:1,5 bis 1:8 eingesetzt. Überschüssiger Alkohol oder überschüssiges Thiol kann gewünschtenfalls nach beendeter Reaktion recycliert werden.

Die Umsetzung erfolgt erfindungsgemäß in Gegenwart eines Phasentransferkatalysators. Als Phasentransferkatalysatoren kommen für das erfindungsgemäße Verfahren wiederum quartäre Ammoniumsalzverbindungen der Formel (V) R₅R₆R₇R₈N⁺A⁻ in Frage, wobei die Reste R₅ bis R₈, sowie A⁻ wie vorne definiert sind.

Bevorzugt sind wiederum Verbindungen der Formel (V) in der R₅-R₈ unabhängig voneinander einen C₁-C₁₆-Alkylrest oder Benzyl und A⁻ Chlorid oder Bromid bedeuten.

Für das erfindungsgemäße Verfahren geeignete Phasentransferkatalysatoren sind aber auch der obigen Beschreibung entsprechende quartäre Phosphoniumsalze, sowie Polyether, cyclischer oder acyclischer Natur, wie PEG's und Kronenether oder Tris-[2-(2-methoxyethoxy)-ethyl]-amin (TDA-1) sowie alkylsubstituierte Guanidiniumsalze.

Bevorzugte Phasentransferkatalysatoren für die Umsetzung der Verbindung der Formel (II) mit der Verbindung der Formel (III) sind Tetrabutylammoniumsalze, Tetrahexylammoniumsalze, Benzyltributylammoniumsalze, Trioctylmethylammoniumsalze, Tridecylmethylammoniumsalze, sowie Methyltributylammoniumsalze oder deren Gemische.
Besonders bevorzugt sind die Chloride und Bromide der oben angeführten Ammoniumsalze.

Die für das erfindungsgemäße Verfahren benötigte Katalysatormenge liegt zwischen 0,1 und 30mol%, bezogen auf die Verbindung der Formel (II), bevorzugt zwischen 0,25 und 25mol% und besonders bevorzugt bei 0,5 bis 15mol%.

Die erfindungsgemäße Umsetzung findet weiters in Anwesenheit einer Base statt. Als Basen eignen sich dabei feste oder gelöste Alkalihydroxide, wie etwa NaOH und KOH, Alkalicarbonate, wie etwa K₂CO₃, Alkalihydrogencarbonate, wie etwa Kaliumhydrogencarbonat, sowie Gemische derselben.
Die Base wird dabei im Überschuss, bezogen auf die Verbindung der Formel (II) eingesetzt.

Gegebenenfalls kann die Umsetzung auch in einem unter den Reaktionsbedingungen basenstabilen Lösungsmittel erfolgen.

Die Reaktion wird bei Reaktionstemperaturen bis zur Rückflusstemperatur durchgeführt, wobei Temperaturen von 60 bis 100°C bevorzugt sind.

Die Reaktionsdauer liegt bevorzugt zwischen 1 h und 24h, besonders bevorzugt bei 4 bis 10h. Längere Reaktionszeiten sind gewünschtenfalls aber auch möglich.

Bei der erfindungsgemäßen Umsetzung wird bevorzugt der Alkohol bzw. das Thiol der Formel (III) zu einem Gemisch aus Verbindung der Formel (II) und Phasentransferkatalysator zugesetzt. Diese Lösung wird sodann mit der Base versetzt und unter Rühren auf die gewünschte Reaktionstemperatur erhitzt.
Nach beendeter Reaktion wird das Reaktionsgemisch filtriert, beispielsweise über eine Glasfritte, die organische Phase abgetrennt und die wässrige Phase gegebenenfalls nach Verdünnen mit Wasser mit einem geeigneten Extraktionsmittel, wie etwa mit Dichlormethan oder dem gegebenenfalls verwendeten Lösungsmittel extrahiert. Der Filterkuchen wird mit dem zur Extraktion verwendeten Lösungsmittel gewaschen und die organischen Phasen werden vereinigt und mit Wasser gewaschen. Abschließend wird das Lösung- bzw. Extraktionsmittel entfernt.

Ein weiterer Gegenstand ist das Gesamtverfahren zur Herstellung der substituierten Pyridin-N-oxid-Verbindungen der Formel (I), das von der Verbindung der Formel (IV) ausgeht.

Durch das erfindungsgemäße Verfahren und das Gesamtverfahren werden die gewünschten substituierten Pyridin-N-oxid-Verbindungen in hoher Selektivität, hoher Ausbeute und hoher Reinheit erhalten.

### Beispiel 1:

397,8g (4,41 mol) 3-Methoxypropanol wurden zu 95g (0,6mol) 4-Chloro-2,3-dimethylpyridin-N-oxid und 17g (0,06mol) Tributylmethylammoniumbromid gegeben. Zu der klaren Lösung wurden 101,4 ml einer 50%igen NaOH tropfenweise zugegeben. Die Reaktionsmischung wurde auf 100°C erhitzt und 8h bei dieser Temperatur gerührt. Die Reaktionsmasse wurde über eine G3-Glasfritte filtriert. Anschließend wurde die organische Phase abgetrennt und die wässrige Phase mit Wasser verdünnt und mehrmals mit Dichlormethan extrahiert. Der Filterkuchen wurde mit Dichlormethan gewaschen und die organischen Phasen vereinigt und mit Wasser gewaschen. Nach dem Entfernen des Lösungsmittels verblieben 101,8g (84,6%) eines braunen Öls, das einen Gehalt von 96,8% an 4-(3-Methoxypropoxy)-2,3-dimethylpyridin-N-oxid aufweist.

### Beispiel 2:

83,0g (0,49mol) 4-Nitro-2,3-dimethylpyridin-N-oxid und 90g (1,54mol) NaCl wurden mit 1350ml CH₃CN, 180ml HCl aq. (36%) and 16,6g Benzyltributylammoniumchlorid versetzt und die erhaltene Suspension wurde unter Rühren 12h lang unter Rückfluss gekocht. Das resultierende Reaktionsgemisch wurde mit 350ml einer 20%igen NaOH auf pH=9 eingestellt, wobei sich der vorhandene Niederschlag größtenteils löste. Die organische Phase wurde abgetrennt, die wässrige Phase bis zur vollständigen Auflösung des Niederschlages verwässert und anschließend mit Dichlormethan extrahiert. Die organischen Phasen wurden vereinigt und das Lösungsmittel im Vakuum entfernt. Es wurden 76,6g (98,5%)4-Chloro-2,3-dimethyl-pyridin-N-oxid erhalten.

## Patentansprüche

1. Verfahren zur Herstellung von substituierten Pyridin-N-oxid-verbindungen der Formel 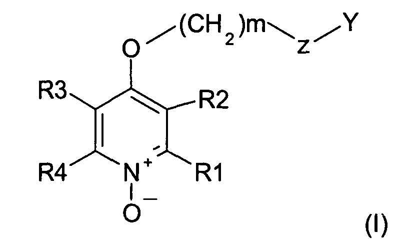 in der R1, R2, R3 und R4 unabhängig voneinander H, eine Carboxylgruppe oder ein C₁-C₁₂-Alkylrest, der gegebenenfalls ein oder mehrere Atome aus der Gruppe N, O, oder S enthalten kann, sein können, oder R1 und R2 und/oder R3 und R4 gemeinsam einen gegebenenfalls substituierten C₄-C₂₀-Alkylenrest bilden können, der ein oder mehrere Atome aus der Gruppe N, O, oder S enthalten kann,
A Benzyl oder eine Gruppe (CH₂)ₘ bedeutet, wobei
m eine ganze Zahl von 1 bis 12 sein kann,
Z₁ und Z₂ unabhängig voneinander O oder S sind und Y H, einen C₁-C₁₂- Alkylrest, der gegebenenfalls ein oder mehrere Atome aus der Gruppe N, O, oder S enthalten kann, einen C₆-C₂₀-Arylrest oder einen C₅-C₂₀-Heterocyclus bedeutet, wobei die Reste gegebenenfalls durch Halogen, C₁-C₆-Alkyl, Nitro, Phenyl oder tert. Amin substituiert sein können,
oder Z₂ und Y gemeinsam einen gegebenenfalls substituierten Ring oder ein gegebenenfalls substituiertes Ringsystem bilden, wobei der Ring oder das Ringsystem ein oder mehrere Atome aus der Gruppe N, O, oder S enthalten kann,
aus dem korrespondierenden 4-Halo-Pyridin-N-oxid der Formel 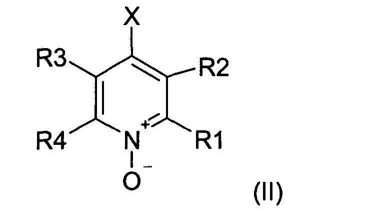 in der R1-R4 wie oben definiert sind und X Chlor, Brom oder Iod bedeutet, **dadurch gekennzeichnet, dass** die Verbindung der Formel (II) in Gegenwart eines Phasentransferkatalysators und einer Base mit einer Verbindung der Formel
HZ₁-A-Z₂-Y (III)
in der Z₁, Z₂, A und Y wie oben definiert sind, bei einer Temperatur bis zur Rückflusstemperatur zu der entsprechenden substituierten Pyridin-N-oxid-Verbindung der Formel (I) umgesetzt wird.

2. Verfahren nach Anspruch 1, **dadurch gekennzeichnet, dass** Verbindungen der Formel (I) hergestellt werden, in der R1, R2, R3 und R4 unabhängig voneinander H oder C₁-C₄-Alkyl bedeuten, A Benzyl oder eine (CH₂)ₘ-Gruppe ist, wobei m eine Zahl von 2 bis 6 bedeutet, Z₁ und Z₂ unabhängig voneinander O oder S sein können und Y H, einen C₁-C₆-Alkylrest, der ein oder zwei Atome aus der Gruppe O oder S enthalten kann, einen C₆-C₁₀-Aryl- oder C₅-C₁₀-Heterocyclusrest bedeutet, wobei die Reste gegebenenfalls durch C₁-C₄-Alkyl, Halogen, Nitro, tert. Amin oder Phenyl substituiert sein können.

3. Verfahren nach Anspruch 1, **dadurch gekennzeichnet, dass** die Verbindungen der Formel (II) und der Formel (III) in einem Molverhältnis von 1:1,1 bis 1:10 eingesetzt werden.

4. Verfahren nach Anspruch 1, **dadurch gekennzeichnet, dass** als Phasentransferkatalysator eine Ammoniumsalzverbindung der Formel (V) R₅R₆R₇R₈N⁺A⁻, in der die Reste R₅ bisR₈ unabhängig voneinander C₁-C₂₀-Alkyl, Phenyl, Arylalkyl oder Alkylaryl bedeuten können, wobei die Reste gegebenenfalls ein- oder mehrfach durch funktionelle Gruppen substituiert sein können und A⁻ ein Anion aus der Gruppe Chlorid, Bromid, Fluorid, lodid, Hydroxid, Hydrogensulfat, Perchlorat, Nitrat, Acetat, Benzoat oder Mesylat bedeutet, oder entsprechende quartäre Phosphoniumsalze, Polyether, cyclischer oder acyclischer Natur, sowie alkylsubstituierte Guanidiniumsalze eingesetzt werden.

5. Verfahren nach Anspruch 1, **dadurch gekennzeichnet, dass** als Phasentransferkatalysator eine Ammoniumsalzverbindung der Formel (V) R₅R₆R₇R₈N⁺A⁻, in der die Reste R₅ bisR₈ unabhängig voneinander C₁-C₁₆-Alkylrest oder einen Benzylrest bedeuten und A- ein Chlorid oder Bromid ist, eingesetzt wird.

6. Verfahren nach Anspruch 1, **dadurch gekennzeichnet, dass** der Phasentransferkatalysator in einer Menge von 0,1 bis 30mol%, bezogen auf die Verbindung der Formel (II) eingesetzt wird.

7. Verfahren zur Herstellung einer 4-Halopyridin-N-oxid-verbindung der Formel (II) 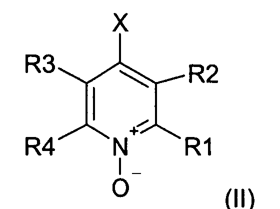 in der der R1, R2, R3 und R4 unabhängig voneinander H, eine Carboxylgruppe oder ein C₁-C₁₂-Alkylrest, der gegebenenfalls ein oder mehrere Atome aus der Gruppe N, O, oder S enthalten kann, sein können, oder R1 und R2 und/oder R3 und R4 gemeinsam einen gegebenenfalls substituierten C₄-C₂₀-Alkylenrest bilden können, der ein oder mehrere Atome aus der Gruppe N, O, oder S enthalten kann, und X Chlor, Brom oder Iod bedeutet, **dadurch gekennzeichnet, dass** eine 4-Nitropyridin-N-oxid-Verbindung der Formel 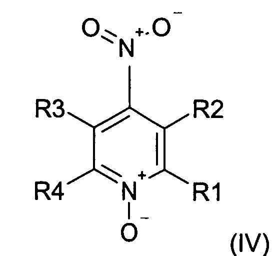 in der R1 bis R4 wie in der Formel (II) definiert sind in Gegenwart eines Phasentransferkatalysators und in Anwesenheit einer Säure mit einem Alkalihalogenid zu der korrespondierenden Verbindungen der Formel (II) umgesetzt wird.

8. Verfahren nach Anspruch 7, **dadurch gekennzeichnet, dass** bei der Umsetzung der Verbindung der Formel (IV) zu der Verbindung der Formel (II) als Phasentransferkatalysator eine Ammoniumsalzverbindung der Formel (V) R₅R₆R₇R₈N⁺A⁻, in der die Reste R₅ bisR₈ unabhängig voneinander C₁-C₂₀-Alkyl, Phenyl, Arylalkyl oder Alkylaryl bedeuten können, wobei die Reste gegebenenfalls ein- oder mehrfach durch funktionelle Gruppen substituiert sein können und A- ein Anion aus der Gruppe Chlorid, Bromid, Fluorid, Iodid, Hydroxid, Hydrogensulfat, Perchlorat, Nitrat, Acetat, Benzoat oder Mesylat bedeutet, oder entsprechende quartäre Phosphoniumsalze, Polyether, cyclischer oder acyclischer Natur, sowie alkylsubstituierte Guanidiniumsalze eingesetzt werden.

9. Verfahren nach Anspruch 7, **dadurch gekennzeichnet, dass** als Phasentransferkatalysator eine Ammoniumsalzverbindung der Formel (V) R₅R₆R₇R₈N⁺A⁻, in der die Reste R₅ bisR₈ unabhängig voneinander C₁-C₁₆-Alkylrest oder einen Benzylrest bedeuten und A⁻ ein Chlorid oder Bromid ist, eingesetzt wird.

10. Verfahren nach Anspruch 7, **dadurch gekennzeichnet, dass** bei der Umsetzung der Verbindung der Formel (IV) zu der Verbindung der Formel (II) als Säure HCl, HBr oder Eisessig verwendet wird

11. Verfahren nach Anspruch 7, **dadurch gekennzeichnet, dass** die Umsetzung der Verbindung der Formel (IV) zu der Verbindung der Formel (II) in einem Lösungsmittel aus der Gruppe der gegebenenfalls halogenierten Kohlenwasserstoffe, Ether oder Nitrile durchgeführt wird.

12. Verfahren zur Herstellung von substituierten Pyridin-N-oxid-verbindungen der Formel 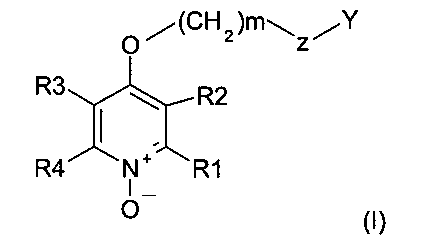 in der R1, R2, R3 und R4 unabhängig voneinander H, eine Carboxylgruppe oder ein C₁-C₁₂-Alkylrest, der gegebenenfalls ein oder mehrere Atome aus der Gruppe N, O, oder S enthalten kann, sein können, oder R1 und R2 und/oder R3 und R4 gemeinsam einen gegebenenfalls substituierten C₄-C₂₀-Alkylenrest bilden können, der ein oder mehrere Atome aus der Gruppe N, O, oder S enthalten kann,
A Benzyl oder eine Gruppe (CH₂)ₘ bedeutet, wobei
m eine ganze Zahl von 1 bis 12 sein kann,
Z₁ und Z₂ unabhängig voneinander O oder S sind und Y H, einen C₁-C₁₂- Alkylrest, der gegebenenfalls ein oder mehrere Atome aus der Gruppe N, O, oder S enthalten kann, einen C₆-C₂₀-Arylrest oder einen C₅-C₂₀-Heterocyclus bedeutet, wobei die Reste gegebenenfalls durch Halogen, C₁-C₆-Alkyl, Nitro, Phenyl oder tert. Amin substituiert sein können,
oder Z₂ und Y gemeinsam einen gegebenenfalls substituierten Ring oder ein gegebenenfalls substituiertes Ringsystem bilden, wobei der Ring oder das Ringsystem ein oder mehrere Atome aus der Gruppe N, O, oder S enthalten kann,
**dadurch gekennzeichnet, dass** eine 4-Nitropyridin-N-oxid-Verbindung der Formel 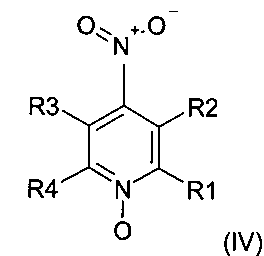 in der R1 bis R4 wie in der Formel (I) definiert sind in Gegenwart eines Phasentransferkatalysators und in Anwesenheit einer Säure mit einem Alkalihalogenid zu der korrespondierenden Verbindungen der Formel 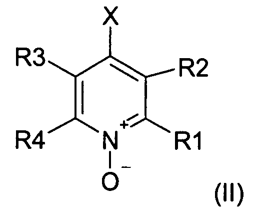 in der R1 bis R4 wie in Formel (I) definiert sind und X Chlor, Brom oder lod bedeutet, umgesetzt wird, worauf die Verbindung der Formel (II) nach Isolierung aus dem Reaktionsgemisch in Gegenwart eines Phasentransferkatalysators und einer Base mit einer Verbindung der Formel
HZ₁-A-Z₂-Y (III)
in der Z₁, Z₂, A und Y wie oben definiert sind, bei einer Temperatur bis zur Rückflusstemperatur zu der entsprechenden substituierten Pyridin-N-oxid-Verbindung der Formel (I) umgesetzt wird.
